# EUROPEAN PATENT APPLICATION

(11) **EP 2 875 839 A1**
(43) Date of publication of application: **27.05.2015**
(21) Application number: 14003999.1
(22) Date of filing: 26.11.2014
(51) Int. Cl.: A61M 16/20, A61M 16/00, A61M 16/01, A61M 39/22

(54) **VALVE MECHANISM**

(30) Priority: 26.11.2013 GB 201320884
(71) Applicant: OES Medical Limited, Witney, Oxfordshire OX29 7QG (GB)
(72) Inventor: Fiedorowicz, Richard, Abingdon, Oxfordshire OX13 6ER (GB)
(74) Representative: Franks & Co (South) Limited

(57) **Abstract**

A control valve (1) for fluids, such as anaesthetic gases, has an inflow duct (18) surrounding an outflow duct (19). A valve diaphragm (13) is moved into and out of contact with a valve seat (20) around a mouth of the outflow duct (19), to open and close the valve (1). The diaphragm (13) is mounted to one end of a shaft (10), which extends through a casing (9) of the valve (1). At a far end of the shaft (10) is mounted a ferrous carrier (3) holding a permanent magnet (2). A coil (4) of electrical wiring is wound around a hollow cylindrical support (5) surrounding the permanent magnet (2). The carrier (3) has a cylindrical ferrous skirt (6) that surrounds the coil (4) in turn. The support (5) and coil (4) are fixed to the casing (9) via a back plate (7) of the valve (1). An electric current is passed through the coil (4), in either direction, generating an electromagnetic field. This interacts with the permanent magnet (2), pushing or pulling the shaft (10) longitudinally, and so displacing the diaphragm (13) into and out of contact with the valve seat (20), operating the valve (1).

## Description

The present invention relates to a valve to control the flow of gases, vapours and other fluids, operated by the interaction of a permanent magnet and an electromagnet. More particularly but not exclusively, it relates to such a valve for use in an anaesthetic ventilator.

Anaesthetic ventilators are used to provide controlled mechanical ventilation when a patient is unable to breathe unaided during surgery. It is important that exactly the desired volume of gas is administered, to produce the required degree of ventilation, without either endangering the patient's health with too high a pressure or volume of gas, or administering an insufficient volume of gas, which will prevent correct patient oxygenation. It is thus important that control elements such as valves within anaesthetic ventilators are accurate, reliable and responsive.

A wide range of valves have been proposed for such purposes; examples are described in International Patent Application No. WO2013/097700, Chinese Utility Model No. CN201921250U; US Patent No. US5215115; US Patent Application No. US2008/262288; and US Patent Application No. US2009/007913. However, these valves do not appear to have been widely adopted.

It is, for example, possible that their relative complexity may discourage their use. It is desirable that such a critical piece of equipment as an anaesthetic ventilator should have a very low risk of in-service failure, and so such valves should be designed to avoid prospective weak points that might fail as a result of prolonged cycles of on/off operation.

It is also desirable that a valve for an anaesthetic ventilator should be as compact as possible.

Additionally, such valves should be responsive in operation and should produce a reliable seal against gas/vapour flow when closed, every time that they are operated.

Such valves would probably also be of benefit in other applications where control of vapour/gas flows is required, and possibly even for the control of liquid flows.

It is hence an object of the present invention to provide a valve for use in an anaesthetic ventilator that obviates the drawbacks of existing valves and which provides at least some, preferably all, of the benefits set out above.

According to a first aspect of the present invention, there is provided a fluid flow control valve for use in an anaesthetic ventilator comprising:
a fluid inflow passage;
a fluid outflow passage;
diaphragm means controllably displaceable between a closed disposition blocking fluid flow between the inflow and outflow passages and an open disposition permitting fluid flow between the inflow and outflow passages; and
a drive mechanism so operatively connected to the diaphragm means as to produce said displacement between its closed and open configurations;
wherein said drive mechanism comprises permanent magnet means fixedly connected to the diaphragm means, and electromagnet means generally encircling said permanent magnet means, such that energising the electromagnet means displaces the permanent magnet means and hence displaces the diaphragm means.

Preferably, the valve comprises valve seat means located between the inflow and outflow passages, such that in said closed disposition, the diaphragm means forms a fluid-tight seal against the valve seat means.

In a preferred embodiment, the valve comprises elongate shaft means mounted adjacent a first end to the diaphragm means and adjacent a second end, remote from the first, to the permanent magnet means.

The shaft means may be provided adjacent its first end with radially-extending plate means, disposed to urge the diaphragm means sealingly against the valve seat means in the closed disposition.

Guide means may be provided to constrain movement of the shaft means, and hence the permanent magnet means and the diaphragm means, to a desired path.

Said guide means may comprise elongate cylindrical bush means coaxially surrounding the shaft means.

Preferably, the permanent magnet means comprises a permanent magnet mounted to a ferrous carrier body, said ferrous carrier body being mounted to the shaft means.

The permanent magnet means may advantageously have a generally cylindrical profile, aligned coaxially with the shaft means.

Preferably, the electromagnet means comprises an electromagnet coil operatively connectable to a source of electrical current.

Control means may be provided, adapted selectively to connect said source of electrical current to the electromagnet coil or to disconnect the source of electrical current therefrom.

Advantageously, the source of electrical current is connectable so as to pass current through the electromagnet coil in either direction.

Preferably, the electromagnet means is substantially surrounded by ferrous housing means.

Said ferrous housing means may be configured to increase a magnetic field produced by the electromagnet means and directed towards the permanent magnet means.

Said ferrous housing means may extend from the ferrous carrier body around the electromagnet means.

Preferably, the electromagnet coil extends around a hollow cylindrical support, mounted to heat sink means.

Said heat sink means may comprise casing means for the drive mechanism of the valve.

Heat generated by passage of current through the electromagnet coil may thus be led away from the inflow and outflow passages and fluids held therein.

Preferably, the diaphragm means comprises a resilient elastomeric material.

The diaphragm means may comprise a displaceable portion connected to the drive mechanism and an anchored portion connected by a flexible portion to the displaceable portion.

Said anchored portion may form a seal, such that the diaphragm means separates the drive mechanism of the valve from the inflow and outflow passages and fluids therein. Preferably, the valve is adapted for use in an anaesthetic ventilator and said fluids comprise a gas containing oxygen gas, with or without an anaesthetic vapour entrained therein.

According to a second aspect of the present invention, there is provided anaesthetic ventilator apparatus containing one or more valves as described in the first aspect above.

An embodiment of the present invention will now be more particularly described by way of example and with reference to the accompanying drawings, in which:
Figure 1 is a cross-sectional elevation of a valve embodying the present invention;
Figure 2 is an exploded isometric view of components of the mechanism of valve of Figure 1; and
Figure 3 is a perspective view of an assembled valve of Figure 1.

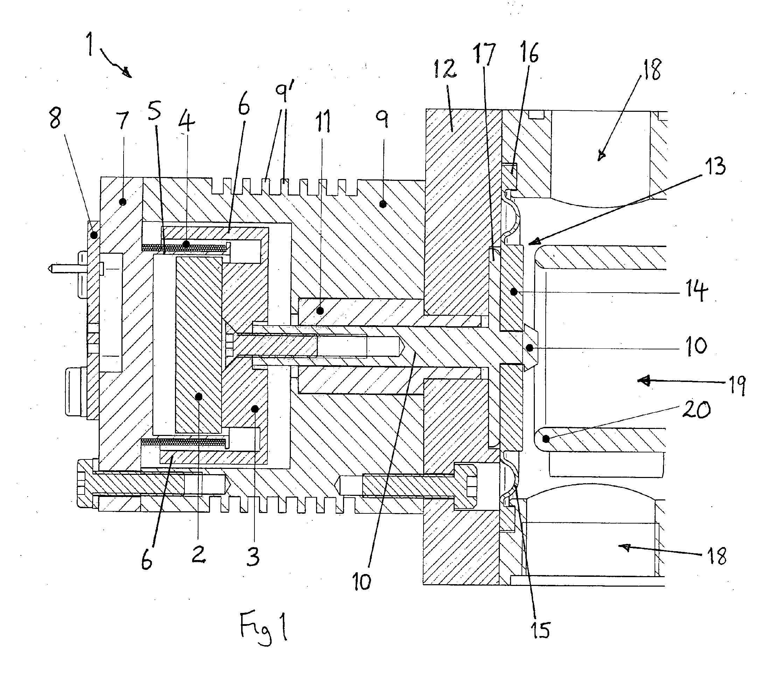

Referring now to the Figures and to Figure 1 in particular, a fluid control valve 1 embodying the present invention comprises a permanent magnet 2 of shallow cylindrical form, mounted to one face of a ferrous metal carrier 3. A coil 4 of electrically-conductive wire is wound around a hollow cylindrical support 5, such that the coil 4 coaxially surrounds the permanent magnet 2. A cylindrical ferrous metal skirt 6 extending from the ferrous metal carrier 3 coaxially surrounds the coil 4 in turn.

The support 5 extends from an inner face of a back plate 7 of the valve 1. A printed circuit board 8 is mounted to an outer face of the back plate 7, and controls operation of the valve 1 as indicated below. A non-ferrous metal casing 9 extends around the permanent magnet 2, carrier 3 and coil 4, and in this embodiment is provided with a plurality of circumferential ribs 9'. The back plate 7, the casing 9 and its ribs 9' will act as a heat sink to dissipate heat generated in the coil 4 when an electrical current is passed therethrough.

The ferrous metal carrier 3 is bolted to a proximal end of an elongate shaft 10. The shaft 10 extends through a cylindrical bush 11 of nylon or other low-friction plastics material, which is supported by the casing 9. The shaft 10 may thus slide longitudinally, guided by the bush 11.

The casing 9 is mounted at its end remote from the back plate 7 to a mounting plate 12. A distal portion of the shaft 10 extends through a corresponding aperture in the mounting plate 12, and a valve diaphragm 13 is mounted adjacent the distal end of the shaft 10.

The valve diaphragm 13 comprises a resilient elastomeric material. The valve diaphragm 13 is generally circular, and comprises a thicker, circular, central sealing portion 14, encircled by a thinner, annular, flexible connecting portion 15, which is in turn encircled by a thicker, annular, anchor portion 16.

A circular pressure plate 17 extends radially outwardly from the shaft 10, between the mounting plate 12 and the valve diaphragm 13. In this embodiment, a recess is provided in the mounting plate 12 to receive the pressure plate 17. The pressure plate 17 corresponds substantially in size and shape to the sealing portion 14 of the valve diaphragm 13. The flexible connecting portion 15 of the valve diaphragm 13 is not only thinner than a remainder of the valve diaphragm 13, but is profiled, as shown. The anchor portion 16 is held securely and sealingly between the mounting plate 12 and a remainder of the valve. Thus, the sealing portion 14 of the valve diaphragm 13 may move orthogonally to a plane of the valve diaphragm 13 and the connecting portion 15 will flex and change shape, while avoiding stress on the anchor portion 16 and reducing strain and fatigue on the connecting portion 15 itself.

The remainder of the valve comprises a fluid inflow duct 18, from which extends a fluid outflow duct 19, in this embodiment in the form of an elongate hollow tube. An end of the fluid outflow duct 19 forms a valve seat 20, located opposite to the sealing portion 14 of the valve diaphragm 13 and having substantially the same shape and diameter.

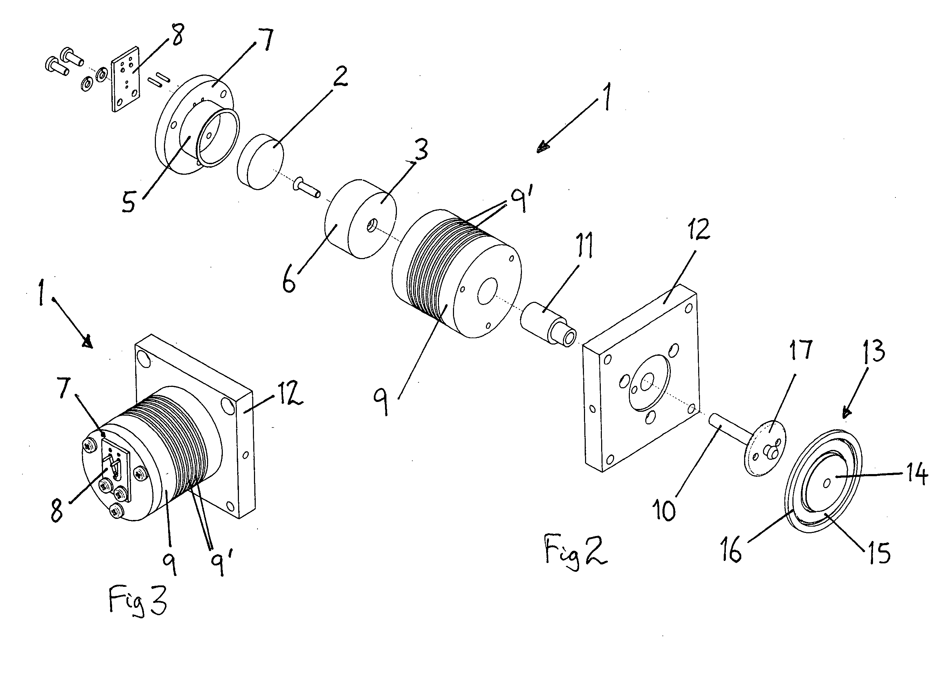

Figure 2 shows an exploded view of the components of the fluid control valve 1 of Figure 1 (with the fluid inflow and outflow ducts 18, 19 omitted for clarity). When these components are assembled, they appear as in Figure 3, forming a compact unit that may easily be mounted to a remainder of the valve by means of the mounting plate 12.

The fluid control valve 1 is operated as follows, starting from an "open valve" configuration as shown in Figure 1. Instructions are sent to the printed circuit board 8, which passes a current through the coil 4, creating an electromagnetic field. The ferrous skirt 6 of the carrier 3, substantially enclosing the coil 4, ensures that the electromagnetic field is concentrated inwardly of the coil 4. Here, it interacts with the magnetic field of the permanent magnet 2, which is arranged so that this interaction urges the permanent magnet 2 distally. The permanent magnet 2, the carrier 3 and the shaft 10 are thus displaced distally. The pressure plate 17 adjacent the distal end of the shaft 10 bears on the sealing portion 14 of the valve diaphragm 13, driving it towards the valve seat 20.

When the sealing portion 14 contacts the valve seat 20, they cooperate to form a seal, blocking passage of fluids from the fluid inflow duct 18 to the fluid outflow duct 19. The pressure plate 17, pressing on the sealing portion 14, ensures that this seal remains fluid-tight. The valve is now in its "closed valve" configuration.

To open the valve, instructions are sent to the printed circuit board 8, which passes a current through the coil 4 in an opposite sense to the current described above. This produces a reversed electromagnetic field that interacts with the field of the permanent magnet 2 to urge the permanent magnet 2 proximally. This retracts the permanent magnet 2, carrier 3 and shaft 10, thus also pulling back the sealing portion 14 of the diaphragm valve 13 and re-opening a passage between the sealing portion 14 and the valve seat 20, allowing fluid to pass into the fluid outflow duct 19 once more. The valve is hence returned to its "open valve" configuration.

The resilience of the connecting portion 15 of the valve diaphragm 13 may also serve to bias the valve diaphragm 13 towards its "open valve" disposition. In some embodiments, this could be used to return the valve to the "open valve" configuration by simply cutting the current to the coil 4 and removing the interaction of magnetic fields that is urging the permanent magnet 2, carrier 3, shaft 10, pressure plate 13 and sealing portion 14 of the valve diaphragm 13 distally. However, this would probably return the valve to its "open valve" configuration more slowly than actively pulling these components back proximally with the reversed electromagnetic field, as described.

The valve mechanism described is compact and straightforward to fit into apparatus such as an anaesthetic ventilator. The opening and closing of the valve is "clean" and positive, responding rapidly to instructions, producing a secure seal, and thus controlling fluid flows, particularly gas or gas/entrained vapour flows, with the accuracy and responsiveness required.

An important benefit of the valve described should be its longevity and lack of "weak points", vulnerable to failure. The structure of the valve diaphragm 13 should permit multiple rapid cycles of operation without significant fatigue or degradation of the elastomer from which it is made.

More significantly, there have been a number of such valves proposed that use the interaction of a permanent magnet and a controllably-energised electromagnet as part of their operating mechanism. However, these appear without exception to locate the permanent magnet in the stationary portion of the valve mechanism, and to locate the electromagnet coil on the moving portion of the mechanism. Such "voice coil" valves have a major problem, in that the constant back-and-forth motion of the coil causes rapid fatigue in the electrical power feeds extending between the stationary portion of the mechanism and the moving coil. As a result, such existing valves are prone to electrical failure, leaving the valve inoperable. The entire apparatus relying on the valve hence fails, with possibly life threatening consequences for apparatus such as an anaesthetic ventilator.

The valves of the present invention thus provide an economical, effective and reliable alternative to existing "voice coil" valves, as well as to other forms of valve that have been proposed in such applications.

## Claims

1. A fluid flow control valve (1) for use in an anaesthetic ventilator, said valve (1) comprising:
a fluid inflow passage (18);
a fluid outflow passage (19);
diaphragm means (13) controllably displaceable between a closed disposition blocking fluid flow between the inflow and outflow passages (18, 19) and an open disposition permitting fluid flow between the inflow and outflow passages (18, 19); and
a drive mechanism so operatively connected to the diaphragm means (13) as to produce said displacement between its closed and open configurations;
**characterised in that** said drive mechanism comprises permanent magnet means (2) fixedly connected to the diaphragm means (13), and electromagnet means (4) generally encircling said permanent magnet means (2), such that energising the electromagnet means (4) displaces the permanent magnet means (2) and hence displaces the diaphragm means (13).

2. A fluid flow control valve (1) as claimed in claim 1, **characterised in that** the valve (1) comprises valve seat means (20) located between the inflow (18) and outflow (19) passages, such that in said closed disposition, the diaphragm means (13) forms a fluid-tight seal against the valve seat means (20).

3. A fluid flow control valve (1) as claimed in claim 2, **characterised in that** the valve (1) comprises elongate shaft means (10) mounted adjacent a first end thereof to the diaphragm means (13) and adjacent a second end thereof, remote from the first, to the permanent magnet means (2).

4. A fluid flow control valve (1) as claimed in claim 3, **characterised in that** the shaft means (10) is provided adjacent its first end with radially-extending plate means (17), disposed to urge the diaphragm means (13) in the closed disposition sealingly against the valve seat means (20).

5. A fluid flow control valve (1) as claimed in either claim 3 or claim 4, **characterised in that** guide means (11) are provided to constrain movement of the shaft means (10), and hence the permanent magnet means (2) and the diaphragm means (13), to a defined path.

6. A fluid flow control valve (1) as claimed in any one of claims 3 to 5, **characterised in that** the permanent magnet means (2) comprises a permanent magnet (2) mounted to a ferrous carrier body (3) said ferrous carrier body (3) being mounted to the shaft means (10).

7. A fluid flow control valve (1) as claimed in any one of the preceding claims, **characterised in that** the electromagnet means (4) comprises an electromagnet coil (4) operatively connectable to a source of electrical current.

8. A fluid flow control valve (1) as claimed in claim 7, **characterised in that** control means (8) are provided, adapted selectively to connect said source of electrical current to the electromagnet coil (4) or to disconnect the source of electrical current therefrom.

9. A fluid flow control valve (1) as claimed in either claim 7 or claim 8, **characterised in that** the source of electrical current is connectable so as to pass current through the electromagnet coil (4) in either direction.

10. A fluid flow control valve (1) as claimed in any one of the preceding claims, **characterised in that** the electromagnet means (4) is substantially surrounded by ferrous housing means (6).

11. A fluid flow control valve (1) as claimed in any one of the preceding claims, **characterised in that** the electromagnet coil (4) extends around a hollow cylindrical support (5), mounted to heat sink means (7, 9, 9').

12. A fluid flow control valve (1) as claimed in any one of the preceding claims, **characterised in that** the diaphragm means (13) comprises a displaceable portion (14) connected to the drive mechanism and an anchored portion (16) connected by a flexible portion (15) to the displaceable portion (14).

13. A fluid control valve (1) as claimed in claim 12, **characterised in that** said anchored portion (16) forms a seal, such that the diaphragm means (13) separates the drive mechanism of the valve (1) from the inflow (18) and outflow (19) passages and fluids therein.

14. A fluid flow control valve (1) as claimed in any one of the preceding claims, **characterised in that** the valve (1) is adapted for use in an anaesthetic ventilator and said fluids comprise a gas containing oxygen gas, with or without an anaesthetic vapour entrained therein.

15. Anaesthetic ventilator apparatus containing one or more fluid flow control valves (1) as claimed in any one of the preceding claims.
